# EUROPEAN PATENT APPLICATION

(11) **EP 4 154 938 A1**
(43) Date of publication of application: **29.03.2023**
(21) Application number: 21807918.4
(22) Date of filing: 20.05.2021
(51) Int. Cl.: A61M 39/10, A61B 5/00, A61M 1/00, A61M 25/00

(54) **BRANCHED CONNECTOR AND CATHETER**

(30) Priority: 21.05.2020 JP 2020088871
(71) Applicant: Tsukada Medical Research Co., Ltd., Tokyo 161-0034 (JP); Osaka University, Suita-shi, Osaka 565-0871 (JP)
(72) Inventor: TSUKADA, Osamu, Tokyo 161-0034 (JP); NAKASA, Akihiko, Tokyo 161-0034 (JP); IGUCHI, Naoya, Suita-shi, Osaka 565-0871 (JP)
(74) Representative: Barker Brettell LLP
(86) International application number: PCT/JP2021/019127
(87) International publication number: WO 2021/235513

(57) **Abstract**

An object is to provide a catheter and a branched connector that can improve sealing properties and have high versatility. In a branched connector 40 used in a catheter 100, the branched connector 40 comprises a first end portion 44 to which a catheter main body 10 is attached and having a first hole portion 43 communicating with the catheter main body 10 being formed at the first end portion 44, and a second end portion 49 including an elastic member and having a second hole portion 48 for inserting a wire-like instrument 20 being formed in the elastic member, and the second hole portion 48 communicates with the first hole portion 43 and has a flow passage area smaller than a flow passage area of the first hole portion 43.

## Description

### TECHNICAL FIELD

The present invention relates to a catheter capable of mainly measuring an oxygen partial pressure and a branched connector used in the catheter.

### BACKGROUND ART

Acute kidney injury (AKI) that occurs in 30 to 50% of inpatients in an intensive care unit adversely affects prognoses of the patients even if the acute kidney injury is mild. A death rate reaches as high as 60% when blood purification is necessary. Transition to chronic renal failure is also a problem. Low oxygen of the renal medulla is one of critical causes of the AKI. It is considered possible to estimate a renal medulla oxygen partial pressure by measuring an oxygen partial pressure in urine in the urinary bladder. In order to measure the oxygen partial pressure in the urine in the urinary bladder, there has been proposed a urethral catheter having an oxygen partial pressure measurement sensor projecting from a side hole portion of the urethral catheter for measuring an oxygen partial pressure in the urinary bladder or the wall of urinary bladder.

The urethral catheter includes a catheter main body, a balloon portion, a branched connector, a sensor portion, and a urination route. The sensor portion for measuring an oxygen partial pressure is inserted into a hole portion formed in the branched connector and pass through the branched connector, and inserted into the catheter main body. In order to prevent a liquid leak in a connecting part of two instruments, there have been proposed a medical instrument for channel switching including a partition wall member having a slit in a syringe connecting part (see, for example, Patent Literature 1) and a medical instrument for guide wire fixing that can surely fix a guide wire even in a high moisture state (see, for example, Patent Literature 2).

### CITATION LIST

### PATENT LITERATURE

PTL 1: Japanese Patent No. 4556701
PTL 2: Japanese Patent No. 5151322

### SUMMARY OF INVENTION

### TECHNICAL PROBLEM

However, in the urethral catheter and the medical instrument according to the related art, when a sensor portion having very small thickness compared with the thickness of the catheter main body such as a wire-like sensor portion for measuring an oxygen partial pressure is used, the sensor portion is not sufficiently sealed and a liquid leak from a position where the sensor portion is inserted into the branched connector is likely to occur.

In the medical instrument for channel switching according to the related art, since the distal end of a syringe is only inserted, an insertion amount is small. When an instrument having a large insertion amount such as the wire-like sensor portion is inserted through the partition wall member, a liquid leak is likely to occur.

An object of the present invention is to provide a catheter and a branched connector that can solve the problems by the related art and improve sealing properties and have high versatility.

### SOLUTION TO PROBLEM

The present invention is a branched connector used in a catheter, the branched connector comprising: a first end portion to which a catheter main body is attached and having a first hole portion communicating with the catheter main body being formed; and a second end portion including an elastic member and having a second hole portion for inserting a wire-like instrument being formed in the elastic member, wherein the second hole portion communicates with the first hole portion and has a flow passage area smaller than a flow passage area of the first hole portion.

In this case, the elastic member may be formed of silicone rubber. The branched connector may include: a main body portion including the first end portion; and a sensor connection port portion including the second end portion, wherein a joint portion for detachably coupling to the main body portion. The main body portion and the sensor connection port portion may be formed of an elastic member, and the joint portion may be formed of a resin material. The joint portion may be formed in a cylindrical shape including a flange portion. The main body portion may be provided with a drainage pipe communicating with the first hole portion and branching in a direction crossing a direction in which the sensor connection port portion is inserted into the main body portion. The sensor connection port portion may be provided with a valve for opening and closing a channel connecting the joint portion and the second end portion. The first end portion may include a cylindrical shape portion for attaching another catheter.

The present invention is a catheter including the above branched connector.

In this case, an outer side of the second end portion may be provided with a transmitting portion for transmitting a detection result by the sensor.

### ADVANTAGEOUS EFFECTS OF INVENTION

The present invention can provide a catheter and a branched connector that can improve sealing properties and have high versatility.

### BRIEF DESCRIPTION OF DRAWINGS

[Fig. 1] Fig. 1 is a schematic diagram showing a state in which a urethral balloon catheter is inserted into a urinary bladder.
[Fig. 2] Fig. 2 is a sectional view schematically showing the distal end of the urethral balloon catheter.
[Fig. 3] Fig. 3 is an enlarged view showing a branched connector of the urethral balloon catheter.
[Fig. 4] Fig. 4 is an enlarged view showing a branched connector according to a modification.
[Fig. 5] Fig. 5 is an enlarged view showing a branched connector according to a modification.
[Fig. 6] Fig. 6 is an enlarged view showing a urethral balloon catheter according to a modification.

### DESCRIPTION OF EMBODIMENTS

A urethral balloon catheter according to an embodiment of the present invention is explained with reference to the drawings.

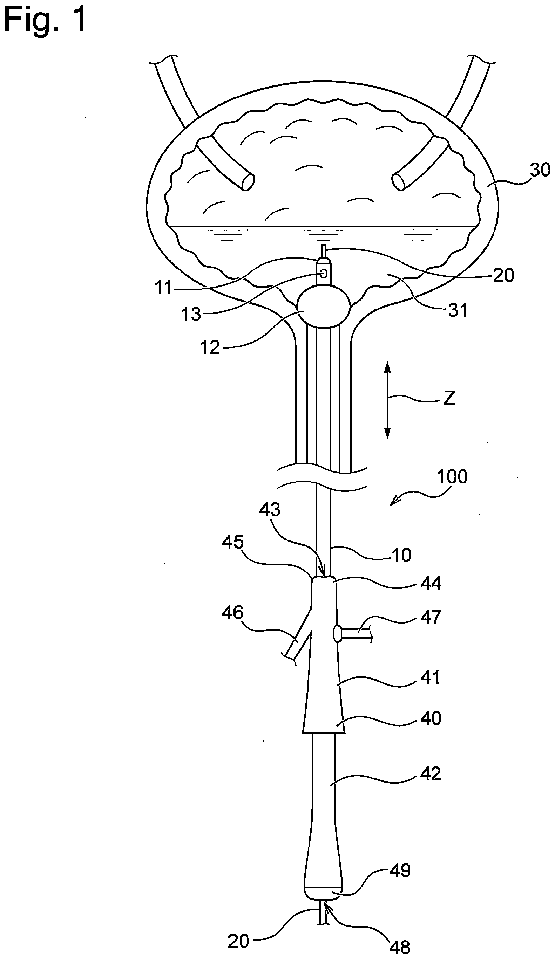

Fig. 1 is a schematic diagram showing a state in which the urethral balloon catheter is inserted into a urinary bladder.

A urethral balloon catheter 100 includes, as shown in Fig. 1, a catheter main body 10, a probe (an instrument) 20, and a branched connector 40.

The catheter main body 10 is formed in a tube shape such that fluid can flow through inside and the probe 20 can be inserted through inside. The catheter main body 10 according to this embodiment is formed of silicone rubber but may be formed of latex, natural rubber, isoprene rubber, thermoplastic resin, for example, PVC and urethan rubber, fluorocarbon rubber and fluorocarbon resin, or the like. A balloon 12 is provided near a distal end 11 of the catheter main body 10. The catheter main body is to be fixed in a urinary bladder 30 by expanding the balloon 12 in a state in which the balloon 12 is inserted into the urinary bladder 30.

The branched connector 40 is formed of an elastic material, for example, silicone rubber but may be formed of latex, natural rubber, isoprene rubber, thermoplastic resin, for example, PVC and urethan rubber, fluorocarbon rubber and fluorocarbon resin, or the like.

A fluorescent optode (optical electrode) that measures the intensity of light emitted from a fluorescent dye reacting with urine 31 in the urinary bladder 30, a double fiber laser doppler probe, and a temperature sensor are incorporated in the probe 20. Consequently, the probe 20 can measure an oxygen partial pressure in urine. Coating may be applied to the probe 20 in order to reduce friction with a lumen of the catheter main body 10. A coating agent only has to be a material that can reduce the friction with the lumen of the catheter main body 10 such as Teflon (registered trademark) coat, Parylene, or hydrophilic polymer. Note that the coating agent explained above may be applied to the lumen side of the catheter main body 10.

The urethral balloon catheter 100 is explained more in detail below.

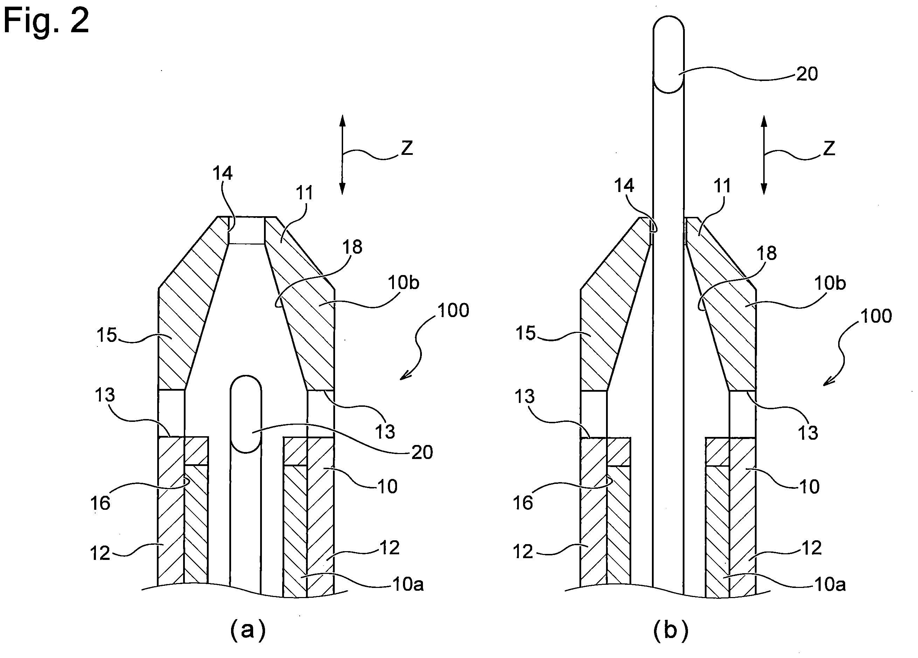

Fig. 2 is a sectional view showing the distal end of the urethral balloon catheter. In Fig. 2, Fig. 2(a) shows a state in which the probe 20 is housed in the catheter main body 10 and Fig. 2(b) shows a state in which the probe 20 projects from the catheter main body.

The catheter main body 10 is configured by combining a tube portion 10a and an insertion end 10b as shown in Fig. 2. In the catheter main body 10, a pair of lateral holes (side holes) 13 for the urine 31 stored in the urinary bladder 30 to flow through is formed near the distal end 11, that is, between the distal end 11 and the balloon 12 (see Fig. 1). The pair of lateral holes (side holes) 13 is located to be opposed and is respectively formed to pass through a wall portion 15 of the catheter main body 10 in a direction perpendicular to an axial direction Z. The pair of lateral holes (side holes) 13 is formed to have a long hole shape expanding in the axial direction Z. Two lateral holes (side holes) 13 are desirably formed. However, the number of the lateral holes (side holes) 13 is not limited to two.

A longitudinal hole (distal hole) 14 is formed at the distal end 11 of the catheter main body 10. The longitudinal hole (distal hole) 14 has a substantially perfect circular shape and is formed to pass through the distal end 11 in the axial direction Z. That is, the longitudinal hole (distal hole) 14 passes through the distal end 11 of the catheter main body 10 in a direction perpendicular to a direction in which the pair of lateral holes (side holes) 13 passes through the catheter main body 10. The longitudinal hole (distal hole) may be a distal end portion formed at an obtuse angle by polishing the distal end portion of the catheter main body, or only the distal end portion may be manufactured by molding and bonded to the catheter main body.

The inner circumference of the longitudinal hole (distal hole) 14 is formed to be the same as or shorter than the inner circumference of a channel 16 passing the inside of the catheter main body 10. That is, a flow passage area of the longitudinal hole (distal hole) 14 is the same as or smaller than a flow passage area of the channel 16. The inner circumference of the longitudinal hole (distal hole) 14 is formed longer than the outer circumference of the probe 20. Consequently, the probe 20 is capable of sliding to pass through the longitudinal hole (distal hole) 14. At this time, in the catheter main body 10, the longitudinal hole (distal hole) 14 may have a substantially perfect circular shape and the inner circumference of the longitudinal hole (distal hole) 14 may have substantially the same length as the outer circumference of the probe. The catheter main body 10 can prevent the probe 20 from moving in a direction perpendicular to the axial direction Z.

The insertion end 10b of the catheter main body 10 includes an inclined portion 18, the inner circumferential surface of which is formed in a conical shape. The inclined portion 18 is formed such that an angle between the inclined portion 18 and the axial direction Z is an acute angle compared with the outer circumferential surface of the insertion end 10b. Consequently, when the probe 20 is pushed in toward the distal end 11, the probe 20 can easily pass through the longitudinal hole 14 while being guided by the inclined portion 18.

In the catheter main body 10, since a gap is formed between the channel 16 and the probe 20, the urine 31 can flow in the gap. Note that, in the catheter main body 10, the channel 16 and the probe 20 may be formed in separate conduits.

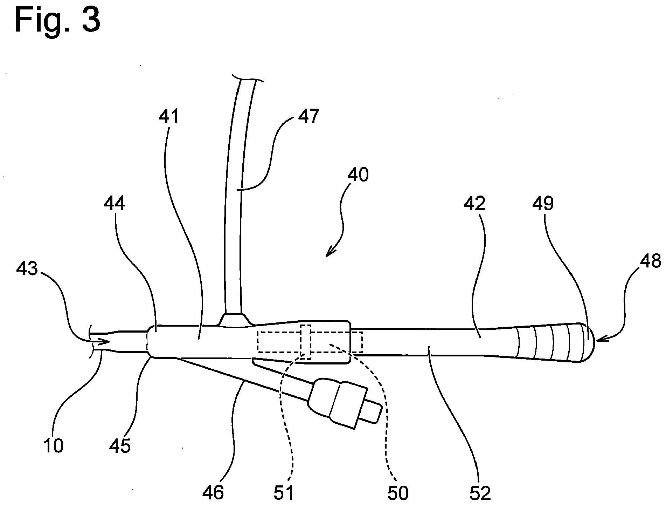

Fig. 3 is an enlarged view showing the branched connector of the urethral balloon catheter.

The branched connector 40 includes, as shown in Fig. 3, a main body portion 41 and a sensor connection port portion 42 detachably coupled to the main body portion 41. Note that, in this embodiment, the main body portion 41 and the sensor connection port portion 42 are detachably provided. However, the main body portion 41 and the sensor connection port portion 42 may be integrally provided.

The main body portion 41 is formed of silicone rubber and includes a first end portion 44 to which the catheter main body 10 is attached, a first hole portion 43 communicating with the catheter main body 10 being formed at the first end portion 44.

An inclined surface 45, an opening portion of which is reduced in a funnel shape toward the first hole portion 43, is formed in the first end portion 44. Consequently, the distal end of the probe 20 inserted into the branched connector 40 is guided toward the first hole portion 43 by the inclined surface 45.

In the main body portion 41, a delivery conduit 46 for delivering gas or liquid such as air or sterilized water to expand the balloon 12 is provided. A one-way valve is equipped in the delivery conduit 46. A syringe can be connected to the one-way valve.

In the main body portion 41, a drainage pipe 47 communicating with the first hole portion 43 and branching in a direction crossing a direction in which the sensor connection port portion 42 is inserted into the main body portion 41, that is, a direction in which the probe 20 is inserted is provided. The drainage pipe 47 may be longer than the entire length of the branched connector 40 and a flow passage area of the conduit may be the same as a flow passage area of the branched connector 40. Consequently, when the urethral balloon catheter 100 is used, it is possible to prevent the catheter main body 10 from being pulled by an instrument or the like connected to the branched connector 40 and discharge urine without being retained in the branched connector 40. Since the drainage pipe 47 is provided in the branched connector 40, the probe 20 can be inserted into the catheter main body 10 through a route not having a bend. A funnel portion (not shown) is provided at the distal end of the drainage pipe 47. A urine storage bag or the like is provided to be connectable to the funnel portion. Consequently, drainage such as urine flowing into the branched connector 40 from the first hole portion 43 through the catheter main body 10 flows out to the urine storage bag or the like from the drainage pipe 47. Note that the drainage pipe 47 according to this embodiment is provided in the main body portion 41 to extend in a direction perpendicular to a direction in which the sensor connection port portion 42 is inserted into the main body portion 41, that is, a direction in which the catheter main body 10 and the probe 20 are inserted. However, not only this, but, like the delivery conduit 46, the drainage pipe 47 may be provided to extend in an oblique direction and a direction away from the first end portion 44 with respect to the direction in which the catheter main body 10 and the probe 20 are inserted of the main body portion 41 .

The sensor connection port portion 42 is mainly formed of silicone rubber and includes a second end portion 49 in which a second hole portion 48 for inserting the wire-like probe 20 is formed. Note that the sensor connection port portion 42 may be formed of ABS resin, polycarbonate, or the like. The second hole portion 48 is formed in the elastic member. In this embodiment, the second hole portion 48 is formed as a circular-shape hole in the center of a sheet made of silicone rubber formed in a circular shape. The second hole portion 48 communicates with the first hole portion 43 and has a flow passage area smaller than a flow passage area of the first hole portion 43. The length of the inner circumference of the second hole portion 48 is larger than the length of the outer circumference of the probe 20 in a state in which the probe 20 is not inserted. That is, when the probe 20 has a circular cross section, the inner diameter of the second hole portion 48 is smaller than the outer diameter of the probe 20. Since the second hole portion 48 is formed in the sheet made of silicon rubber, the second hole portion 48 is provided to be expandable in diameter with the inner circumference extending when the probe 20 is inserted. At this time, since the second hole portion 48 is formed in the circular shape in the center of the sheet made of silicone rubber formed in the circular shape, tensile stress substantially uniformly acts when the second hole portion 48 is expanded in diameter. Therefore, a contact surface with the probe 20 adheres to the probe 20 to substantially uniformly tighten the probe 20 and high sealing properties can be realized. Note that, in this embodiment, the second hole portion 48 is formed in one sheet made of silicone rubber. However, the second hole portion 48 may be formed to pass through each of overlapped two or more sheets made of silicone rubber. The second end portion 49 may be formed in a cylindrical shape and may have a shape expanded in a circular shape like a flange at the end portion of a cylindrical shape portion. Further, in the sensor connection port portion 42 according to this embodiment, nothing is interposed in a channel connecting a joint portion 50 and the second hole portion 48. However, a valve for opening and closing the channel connecting the joint portion 50 and the second hole portion 48 may be provided.

The sensor connection port portion 42 is provided with a joint portion 50 for detachably coupling with the main body portion 41. The joint portion 50 is formed of resin material in a cylindrical shape and integrally includes, near the center in in the axial direction of the cylindrical shape, that is, a direction in which the probe 20 is inserted, a flange portion 51 expanding in a direction perpendicular to the axis. When the flange portion 51 is inserted into the funnel portion having the funnel shape formed in the main body portion 41, the flange portion 51 is inserted while pushing and expanding the funnel portion. Consequently, the sensor connection port portion 42 can be easily inserted into and pulled out from the main body portion 41. The joint portion 50 is fixed, at a certain degree of strength, to a part of the sensor connection port portion 42 formed of silicone rubber and is manufactured using a method such as rim molding.

A flow passage area of the joint portion 50 is smaller than a flow passage area of a body portion 52 of the sensor connection port portion 42 but is larger than a flow passage area of the second hole portion 48. In the branched connector 40, the sensor connection port portion 42 can be detached. The probe 20 can also be integrally detached simply by detaching the sensor connection port portion 42. Therefore, it is possible to facilitate replacement of the probe 20.

In the branched connector 40, the main body portion 41 and the sensor connection port portion 42 are coupled. In a straight state in which the main body portion 41 and the sensor connection port portion 42 are not bent, the first hole portion 43 and the second hole portion 48 of the joint portion 50 are provided to be located on a straight line. Consequently, in the straight state in which the main body portion 41 and the sensor connection port portion 42 are not bent, if the wire-like probe 20 inserted from the second hole portion 48 passes through the joint portion 50, the probe 20 is directly guided by a channel formed in the joint portion 50 toward the first hole portion 43 or the inclined surface 45 formed around the first hole portion 43. When the probe 20 is inserted into the catheter main body 10, it is difficult to insert the probe 20 to the distal end of the catheter main body 10 because of frictional resistance. Therefore, lubricant is used or a guide instrument (not shown) or the like for guiding the probe 20 is used.

The branched connector 40 of the urethral balloon catheter 100 according to this embodiment comprises the first end portion 44 to which the catheter main body 10 is attached and having the first hole portion 43 communicating with the catheter main body 10 is formed; and the second end portion 49 including the elastic member and having the second hole portion 48 for inserting the wire-like probe 20 being formed in the elastic member. The second hole portion 48 communicates with the first hole portion 43 and has the flow passage area smaller than the flow passage area of the first hole portion 43. Consequently, the second hole portion 48 can adhere to an instrument such as the probe 20 inserted into the branched connector 40 to tighten the instrument, and any other instrument can be inserted into the second hole portion 48 if the other instrument is a wire-like instrument having the same degree of thickness. Therefore, it is possible to improve sealing properties on the contact surface with the probe 20 and improve versatility.

The embodiment of the present invention is explained above. However, the embodiment of the invention explained facilitates understanding of the present invention and does not limit the present invention. The present invention can be changed and improved without departing from the gist of the present invention. Equivalents of the present invention are included in the present invention. For example, in the embodiment, the urethral balloon catheter 100 inserted into the urinary bladder 30 is explained. However, the present invention is not limited to this. The present invention may be applied to a catheter used for another site, for example, a catheter inserted into the upper urinary tract.

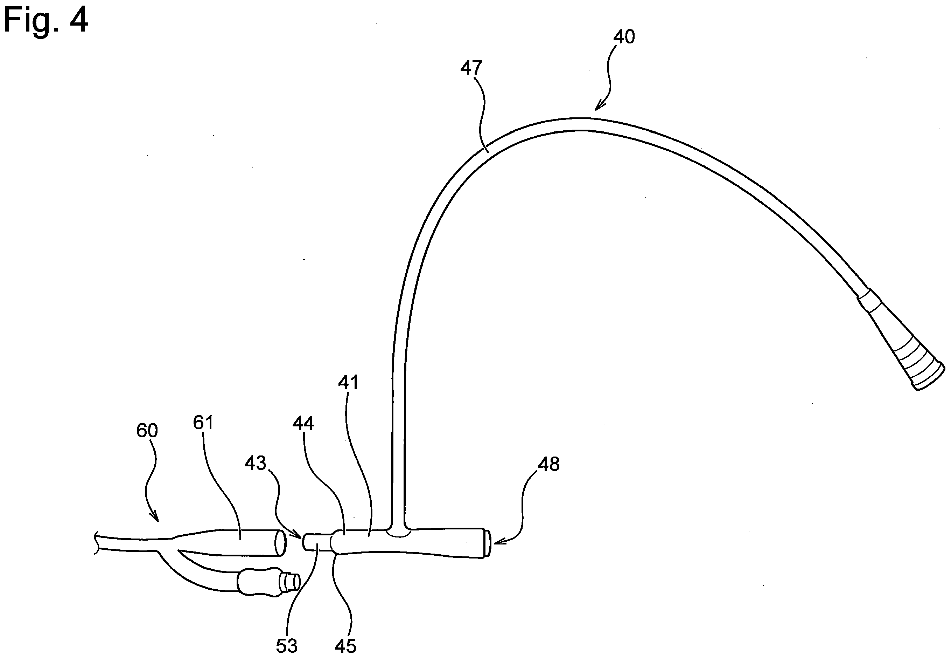

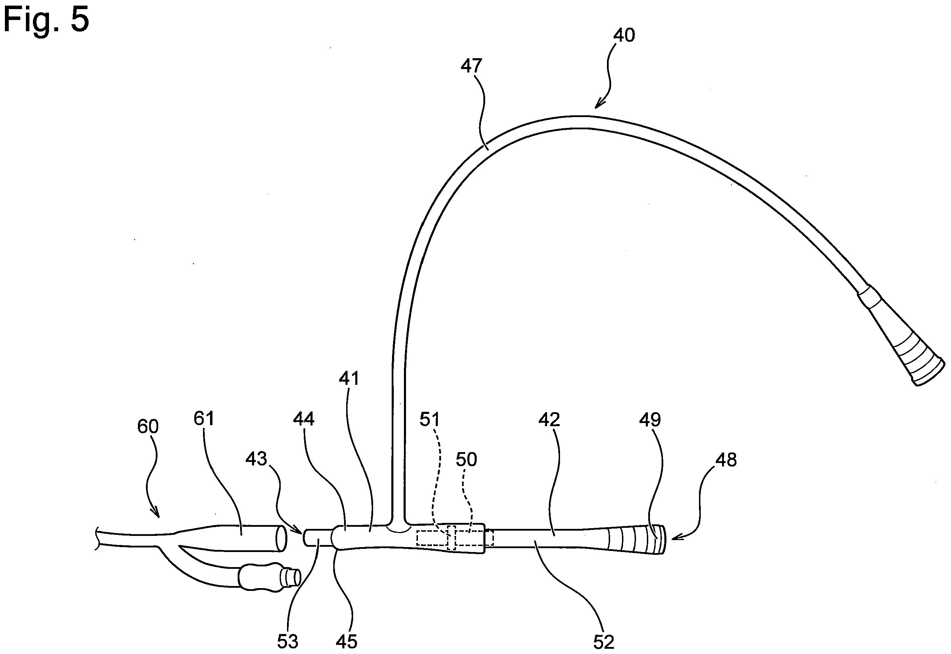

In the embodiment, the urethral balloon catheter 100 in which the catheter main body 10 is bonded to the branched connector 40 in advance is explained. However, not only this, but, for example, as shown in Fig. 4, the first end portion 44 may include a cylindrical shape portion 53 to be inserted into and attached to a funnel portion 61 of commercially available another catheter 60. Consequently, the present invention can be applied to various uses such as a catheter different in a distal end portion structure and thickness. Note that, in Fig. 4, the branched connector 40 is provided as a single member. However, as shown in Fig. 5, the branched connector 40 may include the main body portion 41 and the sensor connection port portion 42 and may be detachably provided.

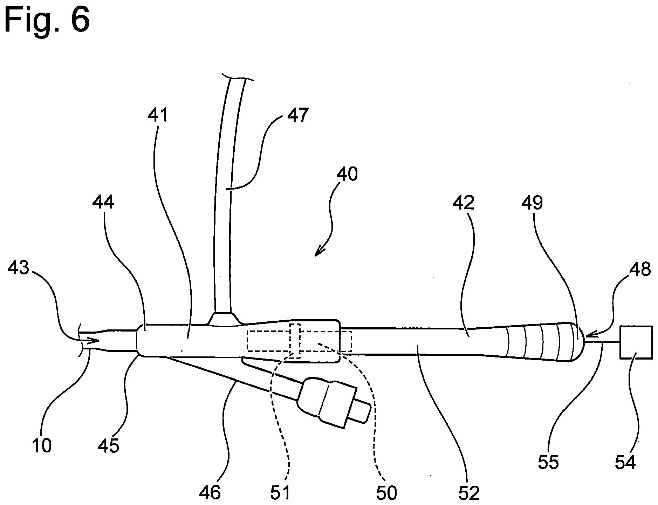

Further, in the embodiment, the probe 20 is connected to measurement equipment by wired connection. However, not only this, but, for example, as shown in Fig. 6, a transmitting portion 54 for transmitting a detection result by the probe 20 to the measurement equipment may be provided at the second end portion 49 via a cable 55. Consequently, since a cable for wired connection is unnecessary, it is possible to enlarge a sphere of activity of a user in a clinical site. When the present invention is applied to a urethral balloon catheter, in view of a situation in a medical site, the urethral balloon catheter is set in a body lower part. However, biological monitor equipment that needs to perform measurement in emergency, an ICU, and the like is often measurement equipment used in the upper half of the body or above. Therefore, in extending an extension cord from the body lower part to a body upper part, disadvantages such as that from a viewpoint of handling of equipment and an increase in an equipment unit price are easily imagined. Therefore, by providing the transmitting portion 54 for data at the end of the branched connector 40 via the cable 55 to make the extension cord unnecessary and providing a data receiving portion in the biological monitor equipment, it is possible to provide a more convenient product.

Furthermore, in the embodiment, a case in which the wire-like probe 20 is inserted into the second hole portion 48 of the urethral balloon catheter 100 is explained. However, not only this, but, an instrument other than the sensor and the like may be inserted into the second hole portion 48 if the instrument is an instrument extending in a wire shape.

### REFERENCE SIGNS LIST

- 10: Catheter main body
- 10a: Tube portion
- 10b: Insertion end
- 11: Distal end
- 12: Balloon
- 14: Longitudinal hole
- 15: Wall portion
- 16: Channel
- 18: Inclined portion
- 20: Probe (instrument)
- 30: Urinary bladder
- 31: Urine
- 40: Branched connector
- 41: Main body portion
- 42: Sensor connection port portion
- 43: First hole portion
- 44: First end portion
- 45: Inclined surface
- 46: Delivery conduit
- 47: Drainage pipe
- 48: Second hole portion
- 49: Second end portion
- 50: Joint portion
- 51: Flange portion
- 52: Body portion
- 53: Cylindrical shape portion
- 54: Transmitting portion
- 55: Cable
- 60: Catheter
- 61: Funnel portion
- 100: Urethral balloon catheter
- Z: Axial direction

## Claims

1. A branched connector used in a catheter, the branched connector comprising:
a first end portion to which a catheter main body is attached and having a first hole portion communicating with the catheter main body being formed; and
a second end portion including an elastic member and having a second hole portion for inserting a wire-like instrument being formed in the elastic member,
wherein, the second hole portion communicates with the first hole portion and has a flow passage area smaller than a flow passage area of the first hole portion.

2. The branched connector according to claim 1, wherein the elastic member is formed of silicone rubber.

3. The branched connector according to claim 1 or 2, further comprising:
a main body portion including the first end portion; and
a sensor connection port portion including the second end portion,
wherein the sensor connection port portion is provided with a joint portion for detachably coupling to the main body portion.

4. The branched connector according to claim 3, wherein the main body portion and the sensor connection port portion are formed of an elastic member, and the joint portion is formed of a resin material.

5. The branched connector according to claim 3 or 4, wherein the joint portion is formed in a cylindrical shape including a flange portion.

6. The branched connector according to any one of claims 3 to 5, wherein the main body portion is provided with a drainage pipe communicating with the first hole portion and branching in a direction crossing a direction in which the sensor connection port portion is inserted into the main body portion.

7. The branched connector according to any one of claims 3 to 6, wherein the sensor connection port portion is provided with a valve for opening and closing a channel connecting the joint portion and the second end portion.

8. The branched connector according to any one of claims 1 to 7, wherein the first end portion includes a cylindrical shape portion for attaching another catheter.

9. A catheter comprising the branched connector according to any one of claims 1 to 8.

10. The catheter according to claim 9, wherein an outer side of the second end portion is provided with a transmitting portion for transmitting a detection result by the sensor.
